(19) Europäisches Patentamt
European Patent Office
Office européen des brevets

(11) Veröffentlichungsnummer: **0 127 808**
**A1**

# (12) EUROPÄISCHE PATENTANMELDUNG

(21) Anmeldenummer: **84105448.9**

(22) Anmeldetag: **14.05.84**

(51) Int. Cl.³: **C 07 C 59/68**
C 07 C 69/712, C 07 C 51/367
C 07 C 67/31, C 07 D 231/10
C 07 C 149/18, A 01 N 39/02
C 07 F 7/08

(30) Priorität: **27.05.83 DE 3319290**

(43) Veröffentlichungstag der Anmeldung:
**12.12.84 Patentblatt 84/50**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Förster, Heinz, Dr.**
**Am Eckbusch 47**
**D-5600 Wuppertal 1(DE)**

(72) Erfinder: **Gallenkamp, Bernd, Dr.**
**Claudiusweg 5**
**D-5600 Wuppertal 1(DE)**

(72) Erfinder: **Priesnitz, Uwe, Dr.**
**Severinstrasse 58**
**D-5650 Solingen(DE)**

(72) Erfinder: **Riebel, Hans-Jochem, Dr.**
**In der Beek 92**
**D-5600 Wuppertal 1(DE)**

(72) Erfinder: **Eue, Ludwig, Dr.**
**Paul-Klee-Strasse 36**
**D-5090 Leverkusen(DE)**

(72) Erfinder: **Schmidt, Robert R., Dr.**
**Im Waldwinkel 110**
**D-5060 Bergisch-Gladbach 2(DE)**

(54) **Optisch aktive Phenoxypropionsäure-Derivate.**

(57) Neue R-Enantiomere von Phenoxypropionsäure-Derivaten der Formel

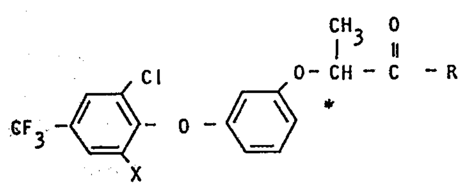

(I)

in welcher

X für Wasserstoff oder Halogen steht, und

R für Hydroxy, Halogen, Alkoxy oder den Rest der Formel

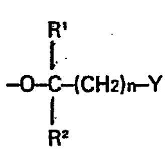

steht,

worin

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxy, Alkoxy-carbonyl oder den rest der Formel

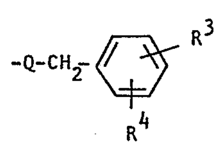

steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

mehrere Verfahren zur Herstellung dieser neuen Stoffe und deren Verwendung als Herbizide.

EP 0 127 808 A1

BAYER AKTIENGESELLSCHAFT 5090 Leverkusen, Bayerwerk

Zentralbereich
Patente, Marken und Lizenzen Dü/Hed-c

I b

## Optisch aktive Phenoxypropionsäure-Derivate

Die Erfindung betrifft neue R-Enantiomere*) von Phenoxypropionsäure-Derivaten, mehrere Verfahren zu deren Herstellung und ihre Verwendung als Herbizide.

Es ist bereits bekannt, daß zahlreiche Phenoxypropionsäure-Derivate herbizide Eigenschaften besitzen (vgl. DE-OS 28 05 981). So kann zum Beispiel das Racemat des 2-[3-(2,6-Dichlor-4-trifluormethyl-phenoxy)-phenoxy]-propionsäure-ethylesters zur Bekämpfung von Unkraut eingesetzt werden. Die Wirkung dieses Stoffes ist gut, jedoch werden bei geringen Aufwandmengen einige Unkräuter nicht immer voll erfaßt.

---

*) Unter R-Enantiomeren sind im vorliegenden Fall jeweils diejenigen optisch aktiven Verbindungen zu verstehen, welche am asymmetrisch substituierten Kohlenstoffatom der Propionsäure-Einheit die R-Konfiguration aufweisen.

Es wurden nun neue R-Enantiomere von Phenoxypropionsäure-Derivaten der Formel

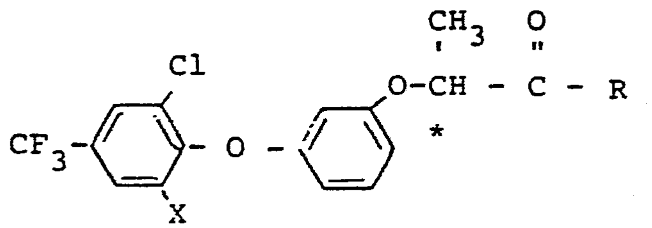

(I)

in welcher

X für Wasserstoff oder Halogen steht, und

R für Hydroxy, Alkoxy, Halogen oder den Rest der Formel

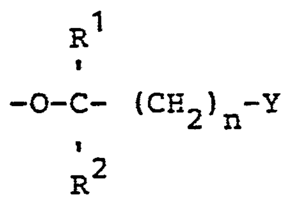

steht,

worin

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxy, Alkoxycarbonyl oder den Rest der Formel

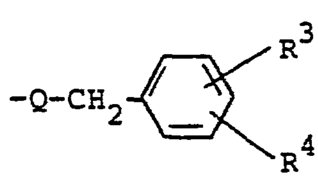

steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

gefunden.

Weiterhin wurde gefunden, daß man die neuen R-Enantiomeren von Phenoxypropionsäure-Derivaten der Formel (I) erhält, wenn man

a) Diphenylether-Derivate der Formel

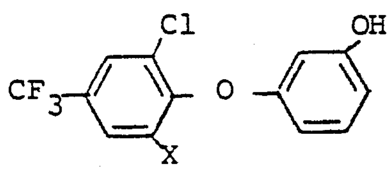

(II)

in welcher

X die oben angegebene Bedeutung hat,

mit S-Enantiomeren der Propionsäure-Derivate der Formel

$$Z - \overset{*}{\underset{}{C}}H(CH_3) - COO - R^5$$

(III)

in welcher

Z für Tosylat oder Mesylat steht und

$R^5$ für Alkyl oder den Rest der Formel

$$-\overset{\displaystyle R^1}{\underset{\displaystyle R^2}{C}}-(CH_2)_n-Y$$ steht,

worin

$R^1$, $R^2$ und n die oben angegebene Bedeutung haben und

Y für Trimethylsilyl, gegebenenfalls substituiertes über Stickstoff gebundenes Azolyl, Alkoxy, Alkoxycarbonyl oder den Rest der Formel

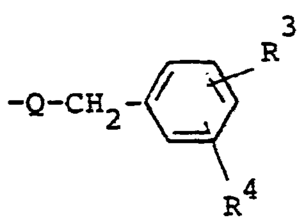

worin

$R^3$,$R^4$ und Q die oben angegebene Bedeutung haben,

gegebenenfalls in Gegenwart eines Säureakzeptors sowie gegebenenfalls in Gegenwart eines Verdünnungsmittels umsetzt und, gegebenenfalls anschließend in Gegenwart eines Verdünnungsmittels verseift und gegebenenfalls dabei entstehende R-Enantiomere von Phenoxypropionsäure-Derivaten der Formel

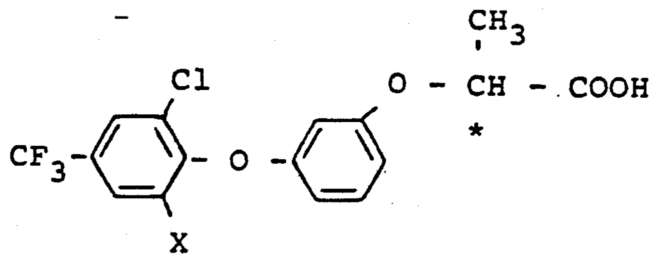

(Ia)

in welcher

X die oben angegebene Bedeutung hat,

durch Halogenierung in die entsprechenden Säurehalogenide überführt,

oder

b) R-Enantiomere von Phenoxypropionsäure-Derivaten der Formel

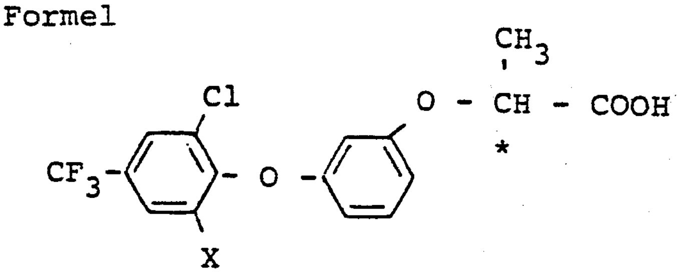

(Ia)

in welcher

X die oben angegebene Bedeutung hat,

α) mit Silylchloriden der Formel

$$\mathrm{Cl-\overset{R^1}{\underset{R^2}{C}}(CH_2)_n-Si(CH_3)_3} \qquad \text{(IV)}$$

in welcher

$R^1$, $R^2$ und n die oben angegebene Bedeutung haben,

oder

ß) mit Verbindungen der Formel

$$Br-\underset{R^2}{\overset{R^1}{C}}-(CH_2)_n-COO-R^6 \qquad (V)$$

in welcher

$R^1$, $R^2$ und n die oben angegebene Bedeutung haben und

$R^6$ für Alkyl steht,

jeweils gegebenenfalls in Gegenwart eines Säurebindemittels sowie gegebenenfalls in Gegenwart eines Verdünnungsmittels umsetzt,

oder

c) R-Enantiomere von Phenoxypropionsäurechloriden der Formel

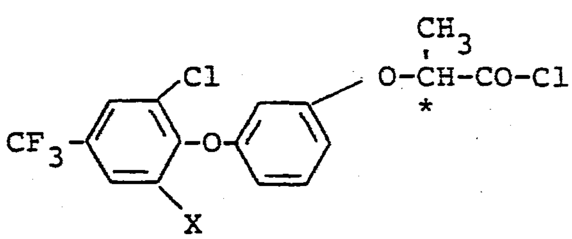

(Ib)

in welcher

0127808

X die oben angegebene Bedeutung hat,

mit Hydroxy-Verbindungen der Formel

$$R^7\text{-OH} \qquad \text{(VI)}$$

in welcher

$R^7$ für Alkyl oder den Rest der Formel

$$\begin{array}{c} R^1 \\ | \\ -C- \\ | \\ R^2 \end{array}\!\!-(CH_2)_n\text{-Y}$$ steht, worin

$R^1$, $R^2$, Y und n die oben angegebene Bedeutung haben,

gegebenenfalls in Gegenwart eines Säureakzeptors sowie gegebenenfalls in Gegenwart eines Verdünnungsmittels umsetzt.

Schließlich wurde gefunden, daß sich die neuen R-Enantiomeren von Phenoxypropionsäure-Derivaten der Formel (I) durch eine hervorragende herbizide Wirksamkeit auszeichnen.

Überraschenderweise besitzen die erfindungsgemäßen R-Enantiomeren der Phenoxypropionsäure-Derivate der Formel (I) wesentlich bessere herbizide Eigenschaften als die Racemate dieser Stoffe. So übertrifft zum Beispiel das R-Enantiomere des 2-/3-(2,6-Dichlor-4-trifluormethyl-phenoxy)-phenoxy/-propionsäure-ethylesters in seiner

herbiziden Wirksamkeit das entsprechende Racemat, das aus dem Stand der Technik als gut wirksames Herbizid bekannt ist.

Die erfindungsgemäßen R-Enantiomeren von Phenoxypropionsäure-Derivaten sind durch die Formel (I) allgemein definiert. In dieser Formel, in der das asymmetrisch substituierte Kohlenstoffatom durch ein (*) gekennzeichnet ist, steht X vorzugsweise für Wasserstoff oder Chlor. R steht vorzugsweise für Hydroxy, Chlor, Brom, Alkoxy mit 1 bis 4 Kohlenstoffatomen oder für den Rest der Formel

$$-O-\overset{R^1}{\underset{R^2}{C}}-(CH_2)_n-Y,$$

worin $R^1$ und $R^2$ unabhängig voneinander für Wasserstoff oder Methyl stehen, n für 0, 1 oder 2 steht und Y vorzugsweise für Trimethylsilyl oder für einen über ein Ringstickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl. Weiterhin steht Y vorzugsweise für Alkoxy mit 1 bis 4 Kohlenstoffatomen, Alkoxycarbonyl mit 1 bis 4 Kohlenstoffatomen im Alkoxyteil oder für den Rest der Formel

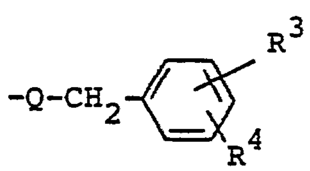

,

worin Q für Sauerstoff, Schwefel, SO oder $SO_2$ steht und

$R^3$ und $R^4$ unabhängig voneinander vorzugsweise für Wasserstoff, Fluor, Chlor, Brom, Iod, Alkyl mit 1 oder 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe stehen.

Diejenigen R-Enantiomeren der Formel (I), in denen $R^1$ für Methyl steht und $R^2$ für Wasserstoff steht, enthalten ein zweites Asymmetriezentrum in der Ester-Einheit. Die betreffenden Stoffe können daher in den folgenden Formen vorliegen:

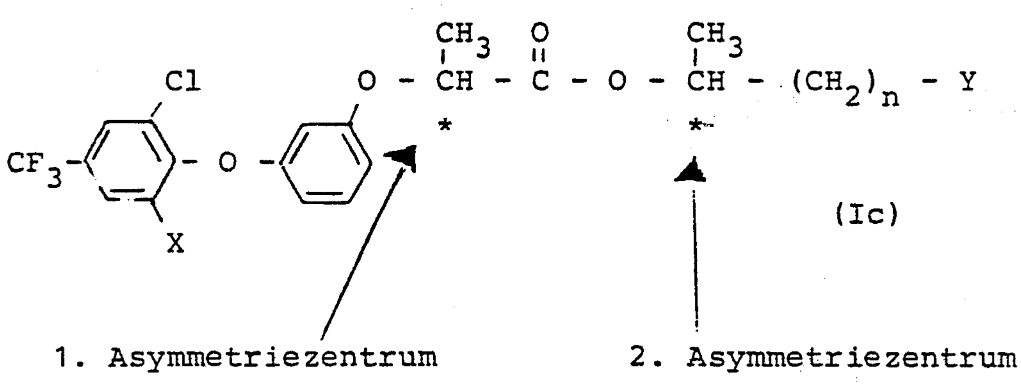

(Ic)

1. Asymmetriezentrum 2. Asymmetriezentrum

| Konfiguration am 1. Asymmetriezentrum | Konfiguration am 2. Asymmetriezentrum |
|---|---|
| R | R |
| R | S |
| R | R/S (Racemat) |

Die vorliegende Erfindung umfaßt sowohl diejenigen Verbindungen, die am zweiten Asymmetriezentrum die R- oder die S-Konfiguration aufweisen, als auch die entsprechenden Racemate.

Verwendet man 2,6-Dichlor-4-trifluormethyl-3'-hydroxy-diphenylether und das S-Enantiomere des 2-Tosyloxy-propionsäure-(ethoxycarbonyl)-methylesters als Ausgangsstoffe, so kann der Verlauf des erfindungsgemäßen Verfahrens (a) durch das folgende Formelschema wiedergegeben werden:

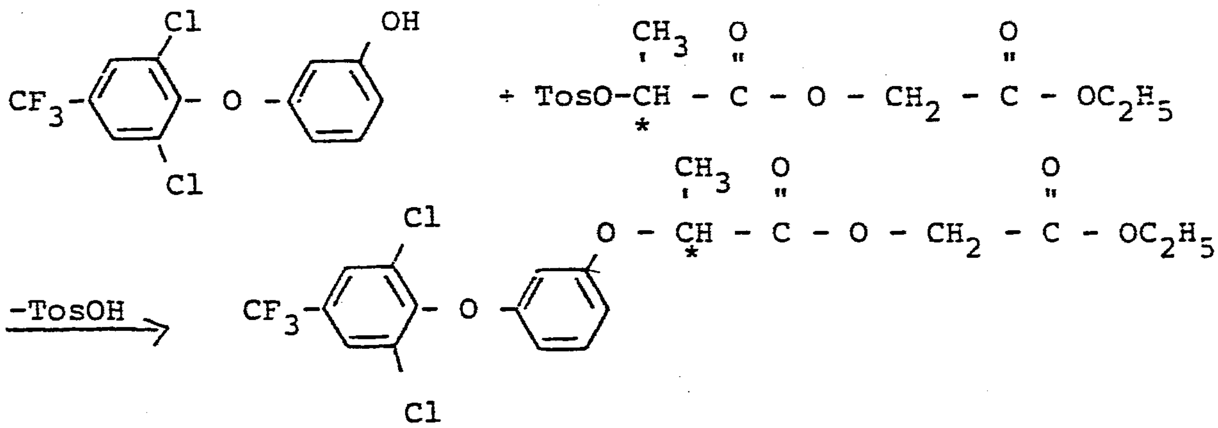


Tos = 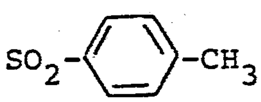 (= Tosyl)

Verwendet man das R-Enantiomere der 2-[3-(2-Chlor-4-trifluormethyl-phenoxy)-phenoxy]-propionsäure und Chlormethyl-trimethyl-silan als Ausgangsstoffe, so kann der Verlauf des erfindungsgemäßen Verfahrens (b, Variante α) durch das folgende Formelschema wiedergegeben werden:

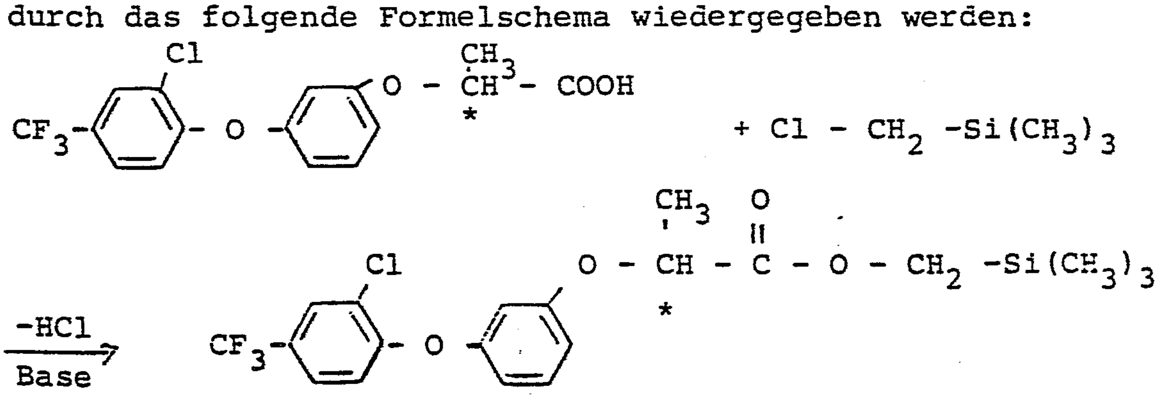

Verwendet man das R-Enantiomere der 2-[3-(2,6-Dichlor-4-trifluormethyl-phenoxy)-phenoxy]-propionsäure und Bromessigsäureethylester als Ausgangsstoffe, so kann der Verlauf des erfindungsgemäßen Verfahrens (b, Variante ß) durch das folgende Formelschema wiedergegeben werden:

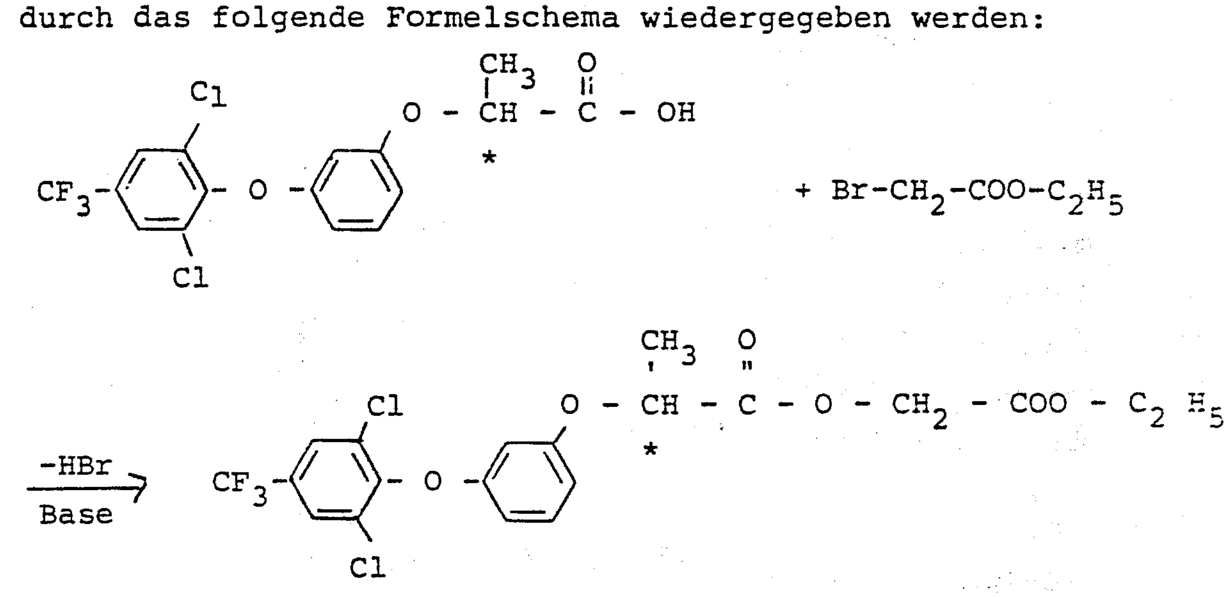


Verwendet man das R-Enantiomere des 2-[3-(2,6-Dichlor-4-trifluormethyl-phenoxy)-phenoxy]-propionsäure-chlorids und 2-(Pyrazol-1-yl)-ethanol als Ausgangsstoffe, so kann der Verlauf des erfindungsgemäßen Verfahrens (c) durch das folgende Formelschema wiedergegeben werden:

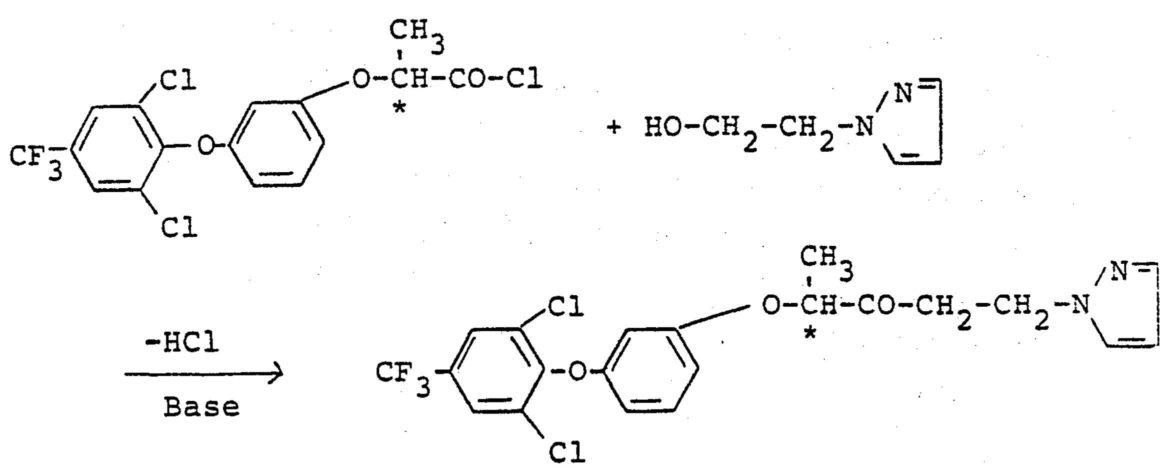

Die bei der Durchführung des erfindungsgemäßen Verfahrens (a) als Ausgangsstoffe benötigten Diphenylether-Derivate sind durch die Formel (II) definiert. In dieser Formel steht X vorzugsweise für Wasserstoff oder Chlor.

Die Diphenylether-Derivate der Formel (II) sind bekannt (vgl. DE-OS 28 05 981).

Die bei dem erfindungsgemäßen Verfahren (a) weiterhin als Ausgangsstoffe benötigten S-Enantiomeren der Propionsäure-Derivate sind durch die Formel (III) definiert. In dieser Formel steht Z für Tosylat ($-O-SO_2-$ 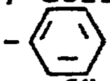 $-CH_3$) oder Mesylat ($-O-SO_2-CH_3$). $R^5$ steht vorzugsweise für Alkyl mit 1 bis 4 Kohlenstoffatomen oder für den Rest der Formel

$$-\overset{R^1}{\underset{R^2}{C}}-(CH_2)_n-Y$$

, worin $R^1$ und $R^2$ unabhängig voneinander für Wasserstoff oder Methyl stehen, n für 0, 1 oder 2 steht und Y vorzugsweise für Trimethylsilyl oder einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl. Weiterhin steht Y vorzugsweise für Alkoxy mit 1 bis 4 Kohlenstoffatomen, Alkoxycarbonyl mit 1 bis 4 Kohlenstoffatomen im Alkylteil oder für den Rest der Formel

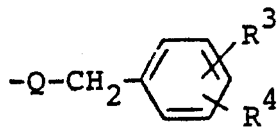 , worin Q für Sauerstoff, Schwefel,

SO oder $SO_2$ steht, und $R^3$ und $R^4$ unabhängig voneinander vorzugsweise für Wasserstoff, Fluor, Chlor, Brom, Iod, Alkyl mit 1 oder 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe stehen.

Diejenigen Stoffe der Formel (III), in denen $R^5$ für den Rest $-\overset{CH_3}{\overset{|}{C}}H-(CH_2)_n-Y$ steht, enthalten im Ester-Teil ein zweites Asymmetrie-Zentrum, das in der R- oder S-Konfiguration vorliegen kann. In den entsprechenden Racematen leistet das asymmetrisch substituierte Kohlenstoffatom im Ester-Teil keinen Beitrag zur optischen Aktivität der S-Enantiomeren der Propionsäure-Derivate der Formel (III).

Die S-Enantiomeren der Propionsäure-Derivate der Formel (III) sind bekannt oder lassen sich nach üblichen Methoden in einfacher Weise herstellen. So erhält man die S-Enantiomeren der Propionsäure-Derivate der Formel (III), indem man S-Enantiomere von Propionylchloriden der Formel

$$Z - \underset{*}{\overset{CH_3}{\overset{|}{C}H}} - CO - Cl \qquad (VII)$$

in welcher

Z die oben angegebene Bedeutung hat,

mit Hydroxyverbindungen der Formel

$$HO - R^5 \qquad (VIII)$$

in welcher

$R^5$ die oben angegebene Bedeutung hat,

gegebenenfalls in Gegenwart eines Säureakzeptors sowie gegebenenfalls in Gegenwart eines Verdünnungsmittels umsetzt.

Die bei der Herstellung der S-Enantiomeren der Propionsäure-Derivate der Formel (III) als Ausgangsstoffe benötigten S-Enantiomeren von Propionylchloriden der Formel (VII) sind bekannt oder lassen sich nach bekannten Methoden herstellen. Die für die Umsetzung zur Synthese der S-Enantiomeren der Propionsäure-Derivate der Formel (III) außerdem als Reaktionskomponenten benötigten Hydroxyverbindungen der Formel (VIII) sind ebenfalls bekannt oder lassen sich nach bekannten Methoden in einfacher Weise herstellen.

Bei diesem Verfahren zur Herstellung der S-Enantiomeren der Propionsäure-Derivate der Formel (III) entsprechen die Umsetzungsbedingungen denen des erfindungsgemäßen Verfahrens (a) (vgl. unten).

Der Einsatz von S-Enantiomeren der Phenoxypropionsäure-Derivate der Formel (III) ist bei dem erfindungsgemäßen Verfahren (a) deshalb erforderlich, weil im Verlauf der

Reaktion eine Walden'sche Umkehr am asymmetrisch substituierten Kohlenstoffatom der Propionsäure-Einheit erfolgt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalihydroxide, Erdalkalihydroxide und -oxide, wie z.B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo-/4,3,0/-non-5-en (DBN), 1,8-Diazabicyclo/5,4,0/-undec-7-en (DBU) und Pyridin.

Als Verdünnungsmittel kommen bei der Durchführung des erfindungsgemäßen Verfahrens (a) alle inerten organischen Solventien in Betracht. Vorzugsweise verwendbar sind aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäuremethylester und - ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Umsetzung nach dem erfindungsgemäßen Verfahren (a) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren.

Ist die Herstellung einer Verbindung der Formel (I) beabsichtigt, in der R für Hydroxy steht, so geht man zweckmäßigerweise so vor, daß man einen nach dem erfindungsgemäßen Verfahren (a) hergestellten Ester (R = Alkoxy) nach üblichen Methoden verseift. Vorzugsweise verwendet man wäßrige Alkalihydroxidlaugen, wie z.B. Natronlauge oder Kalilauge, als Verseifungsreagenzien.

Die Esterverseifung wird im allgemeinen in Gegenwart eines Verdünnungsmittels vorgenommen. Vorzugsweise in Betracht kommen aromatische Kohlenwasserstoffe, wie Toluol oder Xylol, ferner Alkohole, wie Methanol oder Ethanol, außerdem Ether, wie Dioxan, weiterhin Nitrile, wie Acetonitril und auch Gemische aus organischen Solventien und Wasser.

Die Temperaturen können bei der Durchführung der Esterverseifung innerhalb eines größeren Bereiches variiert werden. Im allgemeinen arbeitet man bei Temperaturen zwischen 0°C und 160°C, vorzugsweise zwischen 20°C und 140°C.

Bei der Durchführung der Esterverseifung geht man im allgemeinen so vor, daß man den Ester der Formel (I) in Gegenwart eines Verdünnungsmittels mit einer äquivalenten Menge oder mit einem Überschuß an Base bei der jeweils gewünschten Temperatur behandelt. Die Aufarbeitung erfolgt nach üblichen Methoden. Im allgemeinen geht man so vor, daß man das Reaktionsgemisch durch Abziehen des Lösungsmittels unter vermindertem Druck einengt, den verbleibenden Rückstand in Wasser aufnimmt, mit Mineralsäure, wie zum Beispiel Salzsäure, ansäuert und die sich dabei abscheidende Säure der Formel (I) abtrennt.

Ist die Herstellung einer Verbindung der Formel (I) beabsichtigt, in der R für Halogen steht, so geht man zweckmäßigerweise so vor, daß man eine nach dem erfindungsgemäßen Verfahren (a) hergestellte Säure (R= Hydroxy) mit Halogenierungsmittel, wie zum Beispiel Thionylchlorid, Thionylbromid oder Phosphor-tribromid, gegebenenfalls in Gegenwart eines Katalysators, wie z.B. Dimethylformamid, in Gegenwart eines inerten Verdünnungsmittels, wie zum Beispiel Methylenchlorid oder 1,2-Dichlorethan, bei Temperaturen zwischen 10 und 100°C umsetzt. Die Aufarbeitung erfolgt nach üblichen Methoden.

Die bei dem erfindungsgemäßen Verfahren (b) als Ausgangsstoffe benötigten R-Enantiomeren der Phenoxypropionsäure-Derivate sind durch die Formel (Ia) definiert. In dieser Formel steht X für Wasserstoff oder Chlor.

Die Verbindungen der Formel (Ia) lassen sich aus erfindungsgemäßen Estern der Formel (I), in denen R für Alkoxy steht, in der oben beschriebenen Weise durch Verseifung herstellen.

Die bei dem erfindungsgemäßen Verfahren (b, Variante α) weiterhin als Ausgangsstoffe benötigten Silylchloride sind durch die Formel (IV) definiert. In dieser Formel haben $R^1$, $R^2$ und n vorzugsweise diejenigen Bedeutungen, die bereits im Zusammenhang mit der Beschreibung der erfindungsgemäßen Stoffe der Formel (I) vorzugsweise für diese Reste bzw. für den Index n genannt wurden.

Die Silylchloride der Formel (IV) sind bekannt oder lassen sich in einfacher Weise nach bekannten Methoden herstellen.

Die bei dem erfindungsgemäßen Verfahren (b, Variante ß) weiterhin als Ausgangsstoffe benötigten Verbindungen sind durch die Formel (V) definiert. In dieser Formel haben $R^1$, $R^2$ und n vorzugsweise diejenigen Bedeutungen, die bereits im Zusammenhang mit der Beschreibung der erfindungsgemäßen Stoffe der Formel (I) vorzugsweise für diese Reste bzw. für den Index n genannt wurden. $R^6$ steht vorzugsweise für Alkyl mit 1 bis 4 Kohlenstoffatomen.

Diejenigen Stoffe der Formel (V), in denen $R^1$ für Methyl und $R^2$ für Wasserstoff steht, können entweder als Racemate oder als R- bzw. S-Enantiomere vorliegen. Verwendet man bei dem erfindungsgemäßen Verfahren (b, Variante ß) die R-Enantiomeren dieser Verbindungen der Formel (V), so entstehen diejenigen erfindungsgemäßen Stoffe, die am zweiten Asymmetriezentrum die S-Konfiguration aufweisen, weil im Zuge der Umsetzung eine Walden'sche Umkehr erfolgt. Entsprechend entstehen aus den S-Enantiomeren der Verbindungen der Formel (V) diejenigen erfindungsgemäßen Stoffe, die am zweiten Asymmetriezentrum die R-Konfiguration besitzen. Bei Verwendung von Racematen der Verbindungen der Formel (V) fallen erfindungsgemäße Stoffe an, in denen das asymmetrisch substituierte Kohlenstoffatom im Ester-Teil keinen Beitrag zur optischen Aktivität der Endprodukte leistet.

Die Verbindungen der Formel (V) sind bekannt oder lassen sich in einfacher Weise nach bekannten Methoden herstellen.

Bei der Durchführung des erfindungsgemäßen Verfahrens (b) wird sowohl in der Variante α als auch in der Variante ß vorzugsweise in Gegenwart eines Säureakzeptors und eines Verdünnungsmittels gearbeitet. Hierbei kommen als Säurebindemittel und Verdünnungsmittel vorzugsweise alle diejenigen Stoffe in Betracht, die in diesem Zusammenhang bereits im Falle des erfindungsgemäßen Verfahrens (a) als bevorzugt genannt wurden.

Die Reaktionstemperaturen können auch bei dem erfindungsgemäßen Verfahren (b) sowohl in der Variante α als auch

in der Variante ß innerhalb eines größeren Bereiches variiert werden. Im allgemeinen arbeitet man jeweils bei Temperaturen zwischen -20°C und +160°C, vorzugsweise zwischen 0°C und +140°C.

Das erfindungsgemäße Verfahren (b) wird sowohl in der Variante α als auch in der Variante ß im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b, Variante α und ß) werden die Ausgangsstoffe der Formeln (Ia) und (IV) bzw. (V) im allgemeinen in angenähert äquimolaren Mengen eingesetzt.

Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren.

Die bei dem erfindungsgemäßen Verfahren (c) als Ausgangsstoffe benötigten R-Enantiomeren der Phenoxypropionsäure-chloride sind durch die Formel (Ib) definiert. In dieser Formel steht X für Wasserstoff oder Chlor. Die Verbindungen der Formel (Ib) lassen sich nach dem erfindungsgemäßen Verfahren (a) herstellen (vgl. oben).

Die bei dem erfindungsgemäßen Verfahren (c) weiterhin als Ausgangsstoffe benötigten Hydroxy-Verbindungen sind durch die Formel (VI) definiert. In dieser Formel steht $R^7$ vorzugsweise für Alkyl mit 1 bis 4 Kohlenstoffatomen oder für den Rest der Formel

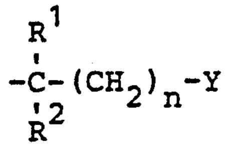 , worin $R^1$ und $R^2$ unabhängig voneinander für Wasserstoff oder Methyl stehen,

n für 0, 1 oder 2 steht und Y vorzugsweise für Trimethylsilyl oder für einen über ein Ringstickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl. Weiterhin steht Y vorzugsweise für Alkoxy mit 1 bis 4 Kohlenstoffatomen, Alkoxycarbonyl mit 1 bis 4 Kohlenstoffatomen im Alkoxyteil oder für den Rest der Formel

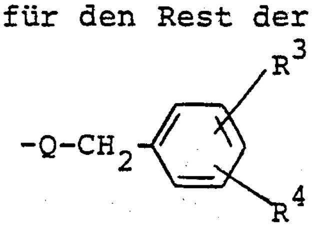 ,

worin Q für Sauerstoff, Schwefel, SO oder $SO_2$ steht und $R^3$ und $R^4$ unabhängig voneinander vorzugsweise für Wasserstoff, Fluor, Chlor, Brom, Iod, Alkyl mit 1 oder 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe stehen.

Die Hydroxy-Verbindungen der Formel (VI) sind bekannt oder lassen sich nach bekannten Verfahren in einfacher Weise herstellen.

Bei der Durchführung des erfindungsgemäßen Verfahrens (c) wird im allgemeinen in Gegenwart eines Verdünnungsmittels sowie in Gegenwart eines Säurebindemittels gearbeitet. Hierbei kommen als Verdünnungsmittel und Säureakzeptoren vorzugsweise alle diejenigen Stoffe in Betracht, die in diesem Zusammenhang bereits im Falle des erfindungsgemäßen Verfahrens (a) als bevorzugt genannt wurden.

Die Reaktionstemperaturen können auch bei dem erfindungsgemäßen Verfahren (c) innerhalb eines größeren Bereiches variiert werden. Im allgemeinen arbeitet man bei Temperaturen zwischen -20°C und +160°C, vorzugsweise zwischen 0°C und +140°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die Ausgangsstoffe der Formeln (Ib) und (VI) im allgemeinen in angenähert äquimolaren Mengen eingesetzt. Es ist jedoch auch möglich, eine der beiden Komponenten in einem größeren Überschuß zu verwenden. Setzt man die Hydroxy-Verbindung in einem großen Überschuß ein, so erübrigt sich in manchen Fällen die Zugabe eines Verdünnungsmittels. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

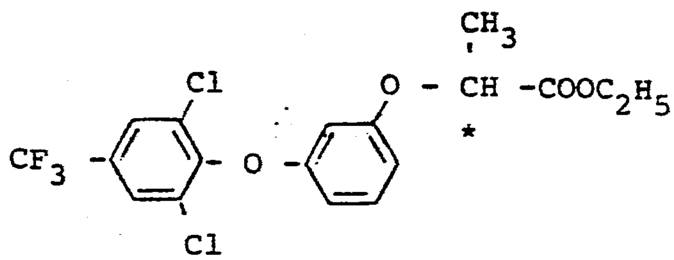

(I - 1)

Ein Gemisch aus 96,9 g (0,3 Mol) 2,6-Dichlor-4-trifluormethyl-3'-hydroxy-diphenylether, 81,7 g (0,3 Mol) des S-Enantiomeren des 2-Tosyloxy-propionsäureethylethers und 82,8 g (0,6 Mol) Kaliumcarbonat in 800 ml Acetonitril wurde 3 Stunden unter Rückfluß erhitzt. Danach wurde aufgearbeitet, indem man das Reaktionsgemisch absaugte, das Filtrat einengte, den verbleibenden Rückstand in Methylenchlorid löste, die entstehende Lösung einmal mit Wasser wusch und nach dem Trocknen unter vermindertem Druck einengte. Der verbleibende Rückstand wurde durch leichtes Erwärmen im Hochvakuum von Resten an flüchtigen Anteilen befreit. Man erhielt auf diese Weise das R-Enantiomere des 2-/3-(2,6-Dichlor-4-trifluormethyl-phenoxy)-phenoxy/-propionsäure-ethylesters in einer Ausbeute von 97 % der Theorie.

Drehwert: $[\alpha]_D^{24}$ = + 1,5 °
(1-molare Lösung in Chloroform; Küvettenlänge 10 cm).

Beispiel 2

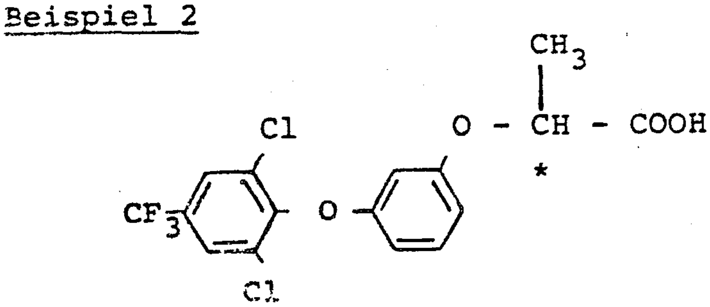

(I - 2)

Eine Lösung von 51,4 g (0,124 Mol) des R-Enantiomeren des 2-[3-(2,6-Dichlor-4-trifluormethyl-phenoxy)-phenoxy]-propionsäure-ethylesters in 95 ml Ethanol wurde unter Rühren bei 0°C in eine Lösung von 5,9 g (0,148 Mol) Natriumhydroxid in 145 ml Wasser eingetropft. Dabei schäumte das Reaktionsgemisch so stark, daß nach Zugabe von 2/3 der Ester-Lösung zunächst noch 10 ml Ethanol in das Reaktionsgemisch gegeben werden mußten, bevor der Rest der Ester-Lösung hinzugefügt werden konnte. Man rührte noch weitere 16 Stunden unter Eiskühlung und arbeitete dann auf, indem man mit Salzsäure ansäuerte, das entstehende Gemisch dreimal mit Methylenchlorid extrahierte und nach dem Trocknen einengte. Der verbliebene Rückstand wurde mit Petrolether gekocht und heiß abgesaugt. Man erhielt auf diese Weise 47,6 g (97,1 % der Theorie) an dem R-Enantiomeren der 2-[3-(2,6-Dichlor-4-trifluormethyl-phenoxy)-phenoxy]-propionsäure in Form einer Festsubstanz vom Schmelzpunkt 120°C.

Drehwert: $[\alpha]_D^{24} = -1,9°$
(1-molare Lösung in Chloroform; Küvettenlänge 10 cm).

Beispiel 3

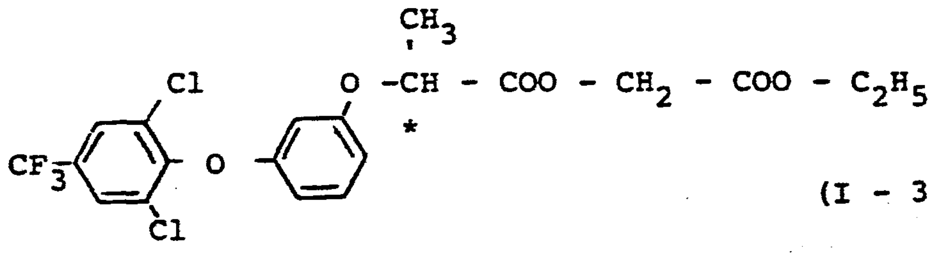

(I - 3)

Ein Gemisch aus 41,0 g (0,104 Mol) des R-Enantiomeren der 2-[3-(2,6-Dichlor-4-trifluormethyl-phenoxy)-phenoxy]-propionsäure, 15,8 g (0,114 Mol) Kaliumcarbonat und 17,4 g (0,104 Mol) Bromessigsäureethylester in 104 ml Acetonitril wurde 1 Stunde unter Rückfluß erhitzt. Danach wurde aufgearbeitet, indem man das Reaktionsgemisch absaugte, das Filtrat einengte, der verbliebenen Rückstand in Methylenchlorid aufnahm, diese Lösung einmal mit Wasser wusch und nach dem Trocknen einengte. Der verbleibende Rückstand wurde durch leichtes Erwärmen im Hochvakuum von Resten an flüchtigen Anteilen befreit. Man erhielt auf diese Weise 34,9 g (70 % der Theorie) an dem R-Enantiomeren des 2-[3-(2,6-Dichlor-4-trifluormethyl-phenoxy)-phenoxy]-propionsäure-(ethoxycarbonyl)-methylesters in Form einer Flüssigkeit mit dem Brechungsindex $n_D^{20}$= 1,5190.

Drehwert: $[\alpha]_D^{24}$ = + 4,68°
(1-molare Lösung in Chloroform; Küvettenlänge 10 cm).

0127808

Beispiel 4

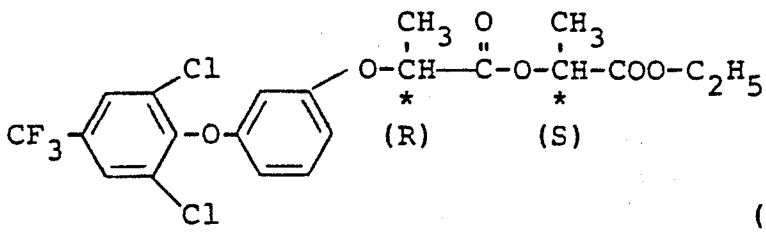

(I-4)

Ein Gemisch aus 16,1 g (0,05 Mol) 2,6-Dichlor-4-trifluormethyl-3'-hydroxy-diphenylether, 8,2 g (0,06 Mol) Kaliumcarbonat, 17,2 g (0,05 Mol) des Tosylates der Formel

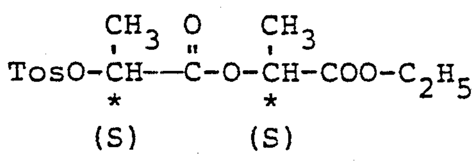

und 100 ml Acetonitril wurde 10 Stunden unter Rückfluß erhitzt. Danach wurde das Reaktionsgemisch abgekühlt und mit 200 ml Wasser versetzt. Das entstehende Gemisch wurde zweimal mit je 100 ml Methylenchlorid extrahiert, und die vereinigten organischen Phasen wurden nach dem Trocknen über Natriumsulfat durch Abziehen des Lösungsmittels unter vermindertem Druck eingeengt. Der verbleibende Rückstand wurde durch Erwärmen im Hochvakuum von Resten an flüchtigen Anteilen befreit. Man erhielt auf diese Weise 17 g (68 % der Theorie) an der Verbindung der Formel (I-4).

$n_D^{20}$ = 1,5292

Drehwert: $[\alpha]_D^{24}$ = - 5,2°

(1-molare Lösung in Chloroform; Küvettenlänge 10 cm).

Beispiel 5

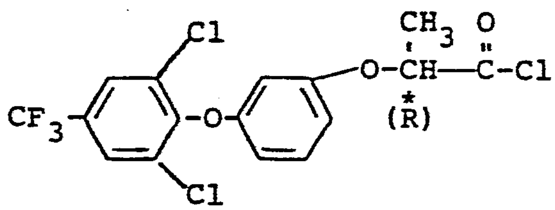

(I-5)

In eine Lösung von 19,7 g (0,05 Mol) des R-Enantiomeren der 2-/3-(2,6-Dichlor-4-trifluormethyl-phenoxy)-phenoxy7-propionsäure in 100 ml Toluol wurden bei 20°C unter Rühren 15 g (0,126 Mol) Thionylchlorid eingetropft. Es wurde 2 Stunden auf 100°C erhitzt. Danach wurde das Reaktionsgemisch durch Abziehen der flüchtigen Anteile unter vermindertem Druck eingeengt. Man erhielt auf diese Weise das R-Enantiomere des 2-/3-(2,6-Dichlor-4-trifluormethyl-phenoxy)-phenyoxy7-propionsäure-chlorids in Form eines öligen Produktes, das zur weiteren Umsetzung verwendet wurde.

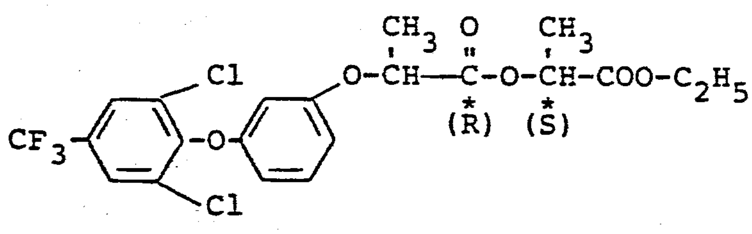

(I-4)

Das in der zuvor beschriebenen Weise hergestellte R-Enantiomere des 2-/3-(2,6-Dichlor-4-trifluormethyl-phenoxy)-phenoxy7-propionsäurechlorids wurde in 50 ml Toluol gelöst und in eine Mischung von 5,5 g (0,055 Mol) Triethylamin und 11,8 g des S-Enantiomeren des Milchsäureethylesters in 100 ml Toluol eingetropft. Es wurde 10

Stunden bei 20°C nachgerührt und dann aufgearbeitet, indem man das Reaktionsgemisch zweimal mit je 100 ml Wasser wusch und danach durch Abziehen des Lösungsmittels unter vermindertem Druck einengte. Der verbleibende Rückstand wurde durch kurzes Erwärmen im Hochvakuum von Resten an flüchtigen Anteilen befreit. Man erhielt auf diese Weise 15 g (60 % der Theorie) an der Verbindung der Formel (I-4).

Drehwert: $[\alpha]_D^{24}$ = - 5,3°
(1-molare Lösung in Chloroform; Küvettenlänge 10 cm).

Nach den in den Beispielen 1 bis 5 angegebenen Methoden wurden auch die in den folgenden Beispielen aufgeführten Stoffe hergestellt.

<u>Beispiel 6</u>

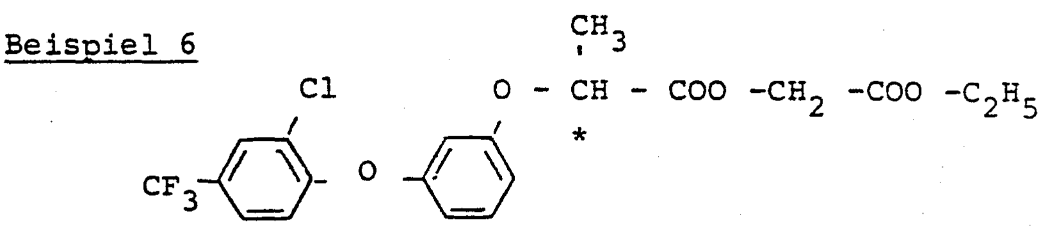

(I-6)

Ausbeute: 77 % der Theorie
Drehwert: $[\alpha]_D^{24}$ = + 6,72.

<u>Beispiel 7</u>

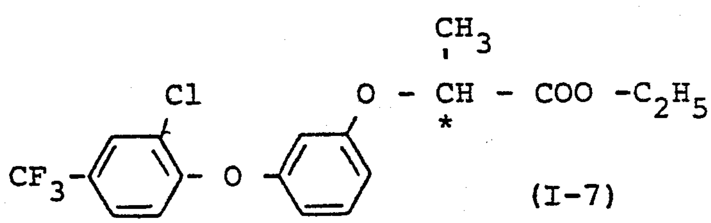

(I-7)

0127808

Ausbeute: 54 % der Theorie
Brechungsindex: $n_D^{20}$ = 1,5171
Drehwert: $[\alpha]_D^{24}$ = +3,65°

Beispiel 8

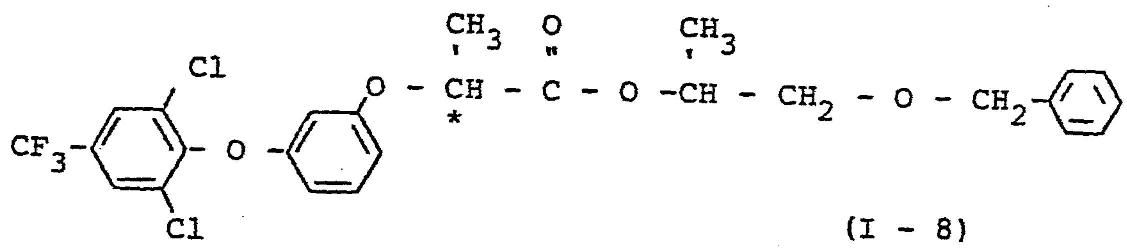

(I - 8)

Ausbeute: 67 % der Theorie
Drehwert: $[\alpha]_D^{24}$ = + 0,16°

Beispiel 9

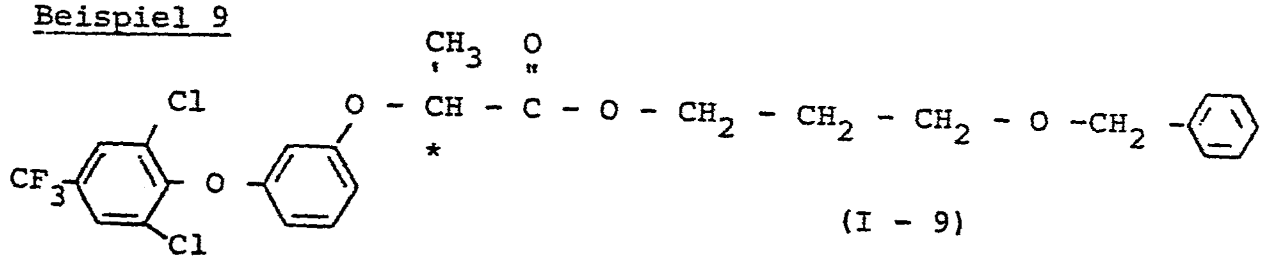

(I - 9)

Ausbeute: 89 % der Theorie
Brechungsindex: $n_D^{20}$ = 1,5380
Drehwert: $[\alpha]_D^{24}$ = 0,89°

Beispiel 10

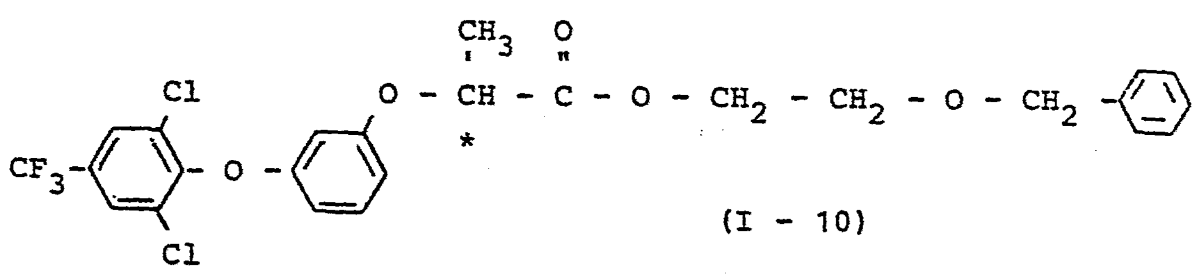

(I - 10)

Ausbeute: 65 % der Theorie
Brechungsindex: $n_D^{20}$ = 1,5415
Drehwert: $[\alpha]_D^{24}$ = + 1,1°

Beispiel 11

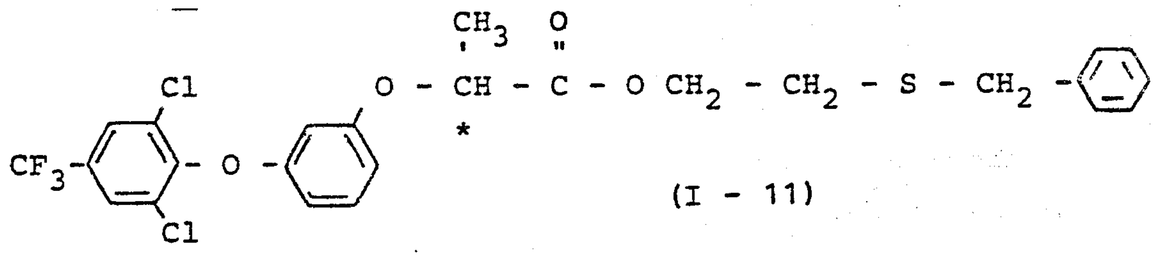

(I - 11)

Ausbeute: 77 % der Theorie
Brechungsindex: $n_D^{20}$ = 1,5554
Drehwert: $[\alpha]_D^{24}$ = + 2,0°

Beispiel 12

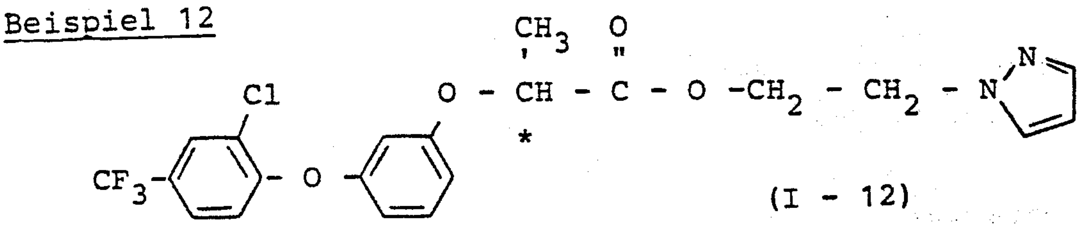

(I - 12)

Ausbeute: 45 % der Theorie
Brechungsindex: $n_D^{20}$ = 1,5065
Drehwert: $[\alpha]_D^{24}$ = 1,6°

Beispiel 13

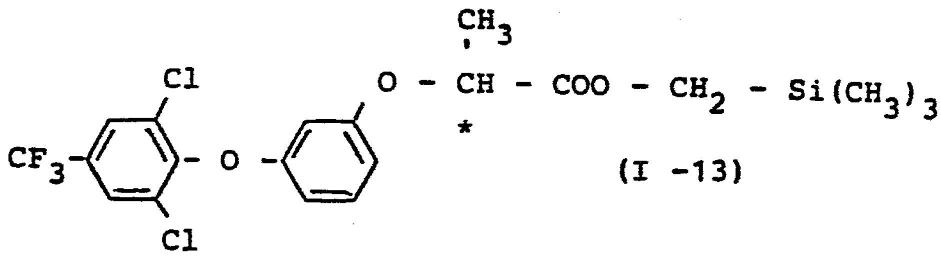

(I -13)

Ausbeute: 68 % der Theorie
Drehwert: $[\alpha]_D^{24}$ = + 4,3°

Beispiel 14

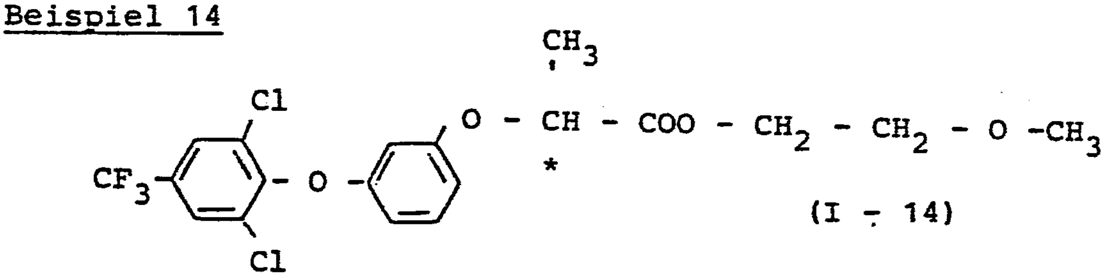

(I - 14)

Ausbeute: 65 % der Theorie
Drehwert: $[\alpha]_D^{24}$ = +1,7°

Beispiel 15

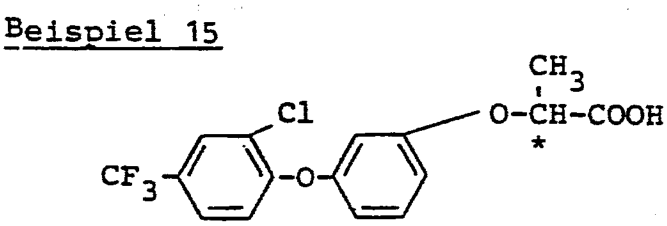

(I - 15)

Ausbeute: 86 % der Theorie
Drehwert: $[\alpha]_D^{24}$ = + 1,52°

Beispiel 16

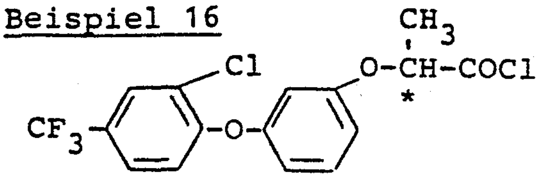

(I-16)

Ausbeute: 90 % der Theorie

Brechungsindex: $n_D^{21}$ = 1,5298

Drehwert: $[\alpha]_D^{24}$ = + 0,37°

Beispiel 17

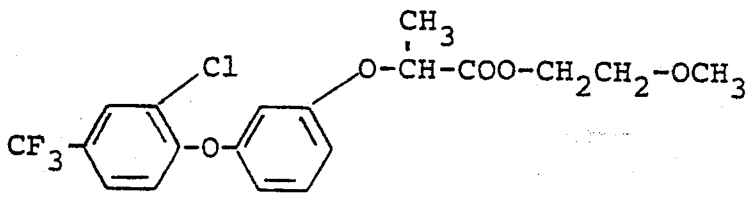

(I-17)

Ausbeute: 74 % der Theorie

Drehwert: $[\alpha]_D^{24}$ = + 3,45°

Beispiel 18

Herstellung des Ausgangsproduktes der Formel:

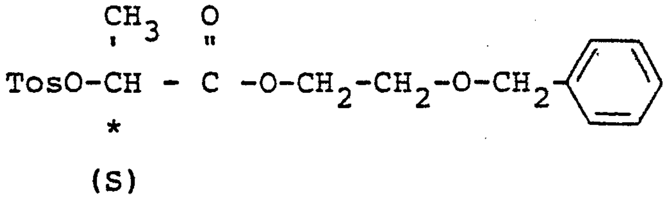

(III - 1)

5,25 g (0,02 Mol) des S-Enantiomeren des Milchsäurechloridtosylates wurden bei 20°C unter Rühren in ein Gemisch aus 3,32 g (0,02 Mol) Glykol-mono-benzylether, 2 g (0,02 Mol) Triethylamin und 50 ml Toluol gegeben. Man rührte weitere 14 Stunden bei 70°C und arbeitete dann auf, indem man das Reaktionsgemisch mit 100 ml Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 6,3 g (80,5 % der Theorie) an S-Enantiomerem des 2-Tosyloxy-propionsäure-(2-benzyloxy)-ethylesters.

Drehwert: $[\alpha]_D^{24}$ = -10,2°
(1-molare Lösung in Chloroform; Küvettenlänge 10 cm).

Nach der in dem Beispiel 18 angegebenen Methode wurden auch die in der folgenden Tabelle formelmäßig aufgeführten Ausgangsstoffe der Formel (III) hergestellt.

Tabelle 1

$$\underset{(S)}{Z-\overset{*}{C}H(CH_3)-COO-R^5} \qquad (III)$$

| Bsp. Nr. | Verbindung | Z | $R^5$ | Drehwert $[\alpha]_D^{24}$ |
|---|---|---|---|---|
| 19 | (III-2) | TosO | 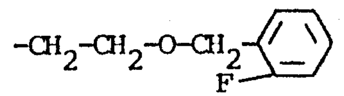 | - 9,7° |
| 20 | (III-3) | TosO | 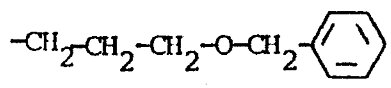 | - 10,3° |
| 21 | (III-4) | TosO | 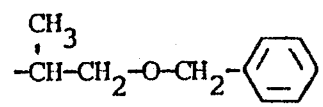 | - 4,1° |
| 22 | (III-5) | TosO | 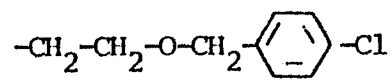 | - 7,6° |
| 23 | (III-6) | TosO | 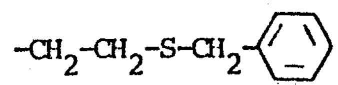 | - 10,9° |
| 24 | (III-7) | TosO | 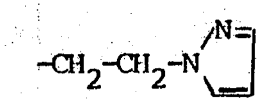 | - 11,4° |
| 25 | (III-8) | TosO | 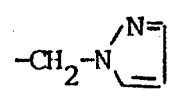 | - 13,7° |

0127808

Tabelle 1 (Fortsetzung)

| Bsp.-Nr. | Verbindung | Z | $R^5$ | Drehwert $[\alpha]_D^{24}$ |
|---|---|---|---|---|
| 26 | (III-9) | TosO | $-\overset{CH_3}{\underset{*}{CH}}-COO-C_2H_5$ (S) | - 17,8° |
| 27 | (III-10) | TosO | $-CH_2-COO-C_2H_5$ | - 11,6° |
| 28 | (III-11) | TosO | $-CH_2-Si(CH_3)_3$ | - 11,4° |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test ein sehr gute herbizide Wirksamkeit.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe (I-7), (I-9), (I-1o), (I-11) und (I-14) zeigen in diesem Test sehr gute herbizide Wirksamkeit zur selektiven Bekämpfung von Galium, Amaranthus und Portulaca in Hafer und Weizen.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

- 0 kein Austrocknen der Blätter, kein Blattfall
- \+ leichtes Austrocknen der Blätter, geringer Blattfall
- ++ starkes Austrocknen der Blätter, starker Blattfall
- +++ sehr starkes Austrocknen der Blätter, sehr starker Blattfall

In diesem Test zeigten die erfindungsgemäßen Stoffe (I-14), (I-15) und (I-17) eine sehr starke Wirksamkeit.

Patentansprüche

1) R-Enantiomere von Phenoxypropionsäure-Derivaten der Formel

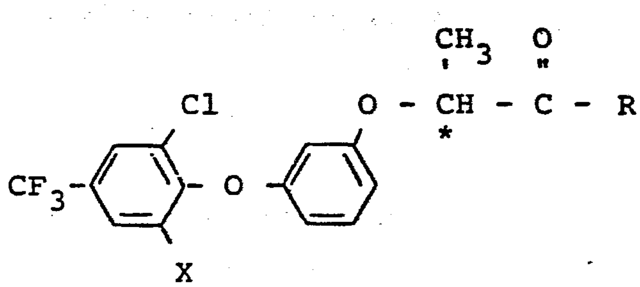

(I)

in welcher

X für Wasserstoff oder Halogen steht und

R für Hydroxy, Alkoxy, Halogen oder den Rest der Formel

$$-O-\overset{R^1}{\underset{R^2}{C}}-(CH_2)_n-Y$$ steht,

worin

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxy, Alkoxycarbonyl oder den Rest der Formel

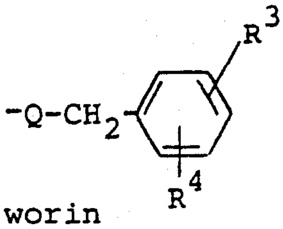 steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.

2) R-Enantiomere von Phenoxypropionsäure-Derivaten der Formel (I), in denen

X für Wasserstoff oder Chlor steht, und

R für Hydroxy, Chlor, Brom, Alkoxy mit 1 bis 4 Kohlenstoffatomen oder für den Rest der Formel

$$-O-\overset{R^1}{\underset{R^2}{C}}-(CH_2)_n-Y$$ steht, worin

$R^1$ und $R^2$ unabhängig voneinander für Wasserstoff oder Methyl stehen,

n für 0, 1 oder 2 steht und

Y für Trimethylsilyl oder für einen über ein Ring-Stickstoffatom gebundenen Pyrazolyl-, Imidazolyl-, 1,2,4-Triazolyl-, 1,2,3-Triazolyl oder 1,3,4-Triazolyl-Rest steht, wobei jeder dieser Azolyl-Reste ein- oder mehrfach, gleichartig oder verschieden substituiert sein kann durch Fluor, Chlor, Brom, Iod, Alkyl mit 1 bis 4 Kohlenstoffatomen, Alkoxy mit 1 bis 4 Kohlenstoffatomen und/oder Phenyl,

und

Y außerdem für Alkoxy mit 1 bis 4 Kohlenstoffatomen, Alkoxycarbonyl mit 1 bis 4 Kohlenstoffatomen im Alkoxyteil steht oder für den Rest der Formel

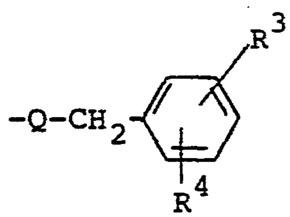

steht,

worin

Q für Sauerstoff, Schwefel, SO oder $SO_2$ steht und

$R^3$ und $R^4$ unabhängig voneinander für Wasserstoff, Fluor, Chlor, Brom, Iod, Alkyl mit 1 oder 2 Kohlenstoffatomen, Alkylthio mit 1 oder 2 Kohlenstoffatomen,

Alkoxy mit 1 oder 2 Kohlenstoffatomen, Nitro, Cyano oder Alkoxycarbonyl mit 1 oder 2 Kohlenstoffatomen in der Alkoxygruppe stehen.

3) Verfahren zur Herstellung von R-Enantiomeren von Phenoxypropionsäure-Derivaten der Formel

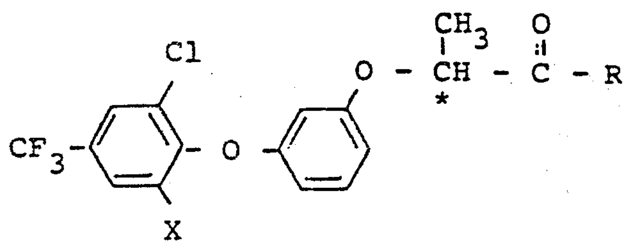

(I)

in welcher

X für Wasserstoff oder Halogen steht und

R für Hydroxy, Halogen, Alkoxy oder den Rest der Formel

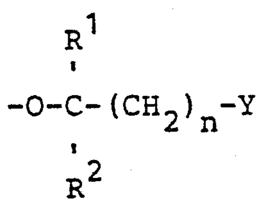

steht,

worin

$R^1$ und $R^2$ unabhängig voneinander für Wasserstoff oder Methyl stehen,

n für 0, 1 oder 2 steht und

Y für Trimethylsilyl, gegebenenfalls substituiertes über Stickstoff gebundenes Azolyl, Alkoxy, Alkoxycarbonyl oder den Rest der Formel

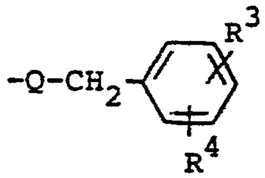

steht,

worin

Q für Sauerstoff, Schwefel, SO oder $SO_2$ steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

dadurch gekennzeichnet, daß man

a) Diphenylether-Derivate der Formel

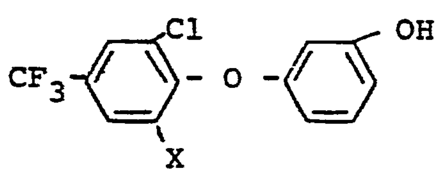

(II)

in welcher

X die oben angegebene Bedeutung hat,

mit S-Enantiomeren der Propionsäure-Derivate der Formel

$$Z-\underset{*}{\overset{CH_3}{CH}}-COO-R^5 \qquad (III)$$

in welcher

Z für Tosylat oder Mesylat steht und

$R^5$ für Alkyl oder den Rest der Formel

$$-\underset{R^2}{\overset{R^1}{C}}-(CH_2)_n-Y \qquad \text{steht,}$$

worin

$R^1$, $R^2$ und n die oben angegebene Bedeutung haben und

Y für Trimethylsilyl, gegebenenfalls substituiertes über Stickstoff gebundenes Azolyl, Alkoxy, Alkoxycarbonyl oder den Rest der Formel

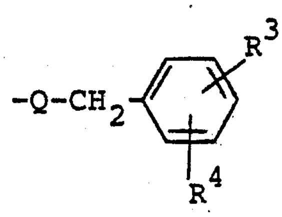

steht,

worin

$R^3$, $R^4$ und Q die oben angegebene Bedeutung haben,

gegebenenfalls in Gegenwart eines Säureakzeptors sowie gegebenenfalls in Gegenwart eines Verdünnungsmittels umsetzt und gegebenenfalls anschließend in Gegenwart eines Verdünnungsmittels verseift und gegebenenfalls dabei entstehende R-Enantiomere von Phenoxypropionsäure-Derivaten der Formel

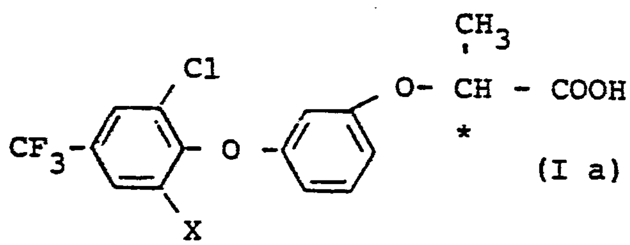

(I a)

in welcher

X die oben angegebene Bedeutung hat,

durch Halogenierung in die entsprechenden Säurehalogenide überführt,

oder

b) R-Enantiomere von Phenoxypropionsäure-Derivaten der Formel

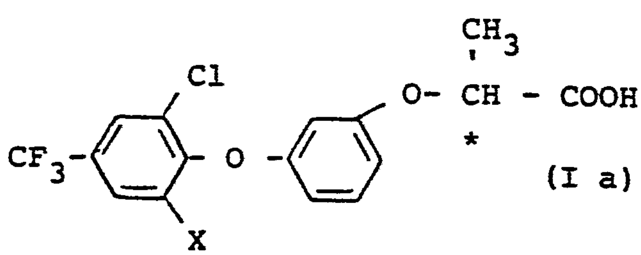

(I a)

in welcher

X die oben angegebene Bedeutung hat,

α) mit Silylchloriden der Formel

$$Cl-\underset{R^2}{\overset{R^1}{C}}-(CH_2)_n-Si(CH_3)_3 \qquad (IV)$$

in welcher

$R^1$, $R^2$ und n die oben angegebene Bedeutung haben,

oder

ß) mit Verbindungen der Formel

$$Br-\underset{R^2}{\overset{R^1}{C}}-(CH_2)_n-COO-R^6 \qquad (V)$$

in welcher

$R^1$, $R^2$ und n die oben angegebene Bedeutung haben und

$R^6$ für Alkyl steht,

jeweils gegebenenfalls in Gegenwart eines Säurebindemittels sowie gegebenenfalls in Gegenwart eines Verdünnungsmittels umsetzt,

oder

c) R-Enantiomere von Phenoxypropionsäurechloriden der Formel

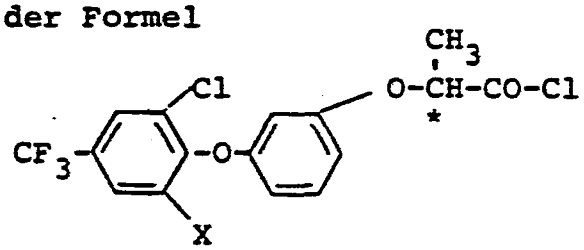

(Ib)

in welcher

X die oben angegebene Bedeutung hat,

mit Hydroxy-Verbindungen der Formel

$$R^7 - OH \qquad (VI)$$

in welcher

$R^7$ für Alkyl oder den Rest der Formel

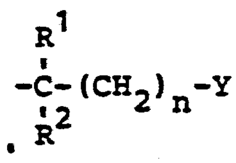

steht, worin

$R^1$, $R^2$, Y und n die oben angegebene Bedeutung haben,

gegebenenfalls in Gegenwart eines Säureakzeptors sowie gegebenenfalls in Gegenwart eines Verdünnungsmittels umsetzt.

4) Herbizide Mittel, gekennzeichnet durch einen Gehalt an mindestens einem R-Enantiomeren eines Phenoxypropionsäure-Derivates der Formel (I).

5) Verwendung von R-Enantiomeren von Phenoxypropionsäure-Derivaten der Formel (I) als Herbizide.

6) Verfahren zur Bekämpfung von Unkräutern, dadurch gekennzeichnet, daß man R-Enantiomere von Phenoxypropionsäure-Derivaten der Formel (I) auf die Unkräuter und/oder deren Lebensraum ausbringt.

7) Verfahren zur Herstellung von herbiziden Mitteln, dadurch gekennzeichnet, daß man R-Enantiomere von Phenoxypropionsäure-Derivaten der Formel (I) mit Streckmitteln und/oder oberflächenaktiven Stoffen vermischt.

8) R-Enantiomeres Phenoxypropionsäure-Derivat der Formel

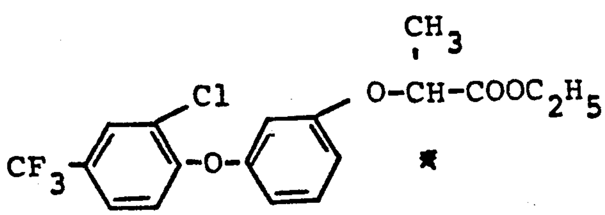

(R)

9) R-Enantiomeres Phenoxypropionsäure-Derivat der Formel

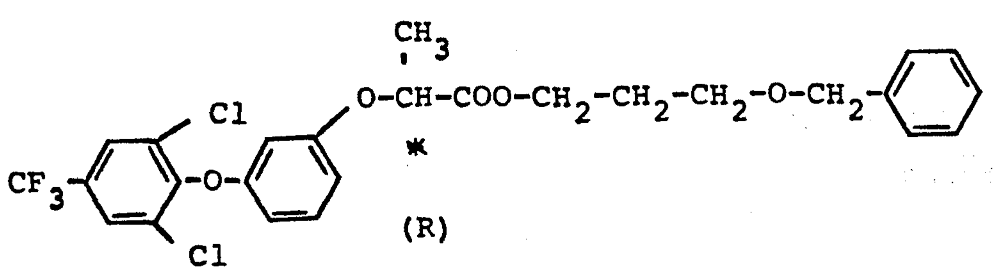

(R)

1o) R-Enantiomeres Phenoxypropionsäure-Derivat der Formel

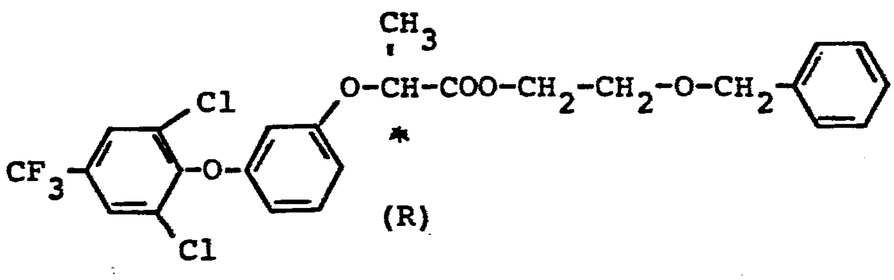

Europäisches Patentamt

# EUROPÄISCHER RECHERCHENBERICHT

0127808
Nummer der Anmeldung

EP 84 10 5448

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | EP-A-0 003 584 (BAYER)<br>* Ansprüche 1-7 *<br>--- | 1-8 | C 07 C 59/68<br>C 07 C 69/712<br>C 07 C 51/367<br>C 07 C 67/31<br>C 07 D 231/10<br>C 07 C 149/18<br>A 01 N 39/02<br>C 07 F 7/08 |
| Y | EP-A-0 025 079 (HOFFMANN-LA ROCHE)<br>* Ansprüche 1-12 *<br>--- | 1,2,4,5 | |
| A | EP-A-0 009 285 (AKZO)<br>* Ansprüche 1-3 *<br>----- | 1,4-6 | |
| | | | **RECHERCHIERTE SACHGEBIETE (Int. Cl. 3)** |
| | | | C 07 C 59/00<br>C 07 C 69/00<br>C 07 C 51/00 |

Der vorliegende Recherchenbericht wurde für alle Patentansprüche erstellt.

| Recherchenort | Abschlußdatum der Recherche | Prüfer |
|---|---|---|
| DEN HAAG | 31-08-1984 | KLAG M.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument